# EUROPEAN PATENT APPLICATION

(11) **EP 1 820 804 A1**
(43) Date of publication of application: **22.08.2007**
(21) Application number: 06003440.2
(22) Date of filing: 20.02.2006
(51) Int. Cl.: C07H 21/00

(54) **Lipidated oligonucleotides**

(71) Applicant: Humboldt-Universität zu Berlin, D-10099 Berlin (DE); Universität Leipzig, 04109 Leipzig (DE)
(72) Inventor: Herrmann, Andreas, 13156 Berlin (DE); Liebscher, Jürgen, 15366 Neuenhagen (DE); Huster, Daniel, 04275 Leipzig (DE); Kurz, Anke, 16341 Panketal (DE); Bunge, Andreas, 06188 Landsberg (DE); Kaczmarek, Oliver, 10319 Berlin (DE); Flasche, Wolfgang, 82327 Tutzing (DE)
(74) Representative: Engelhard, Markus

(57) **Abstract**

The present invention relates to lipidated oligonucleotides, methods of synthesis and uses thereof.

## Description

The present invention relates to lipidated oligonucleotides, methods of synthesis and uses thereof.

Lipidated R NA and DNA oligonucleotides have emerged a s attractive chimeric molecules for various applications in nanobiotechnology, in cell biology and for the development of therapeutic strategies in medicine.^{[1-5]} For example, lipidated oligonucelotides have been synthesised to improve cellular uptake of antisense single-stranded DNA (ssDNA) and interference RNA (iRNA).^{[2;6;7]} Recently, efficient silencing of an endogenous apolipoprotein B in mice has been reported upon injection of short interfering RNAs (siRNAs) coupled to cholesterol.^{[8]} Anchoring in the cell membrane is one of the important processes of membrane passage provided by the lipophilic cholesterol group. For a wider application of such structures, lipid-anchoring oligonucleotides should meet various requirements, which include easy and flexible synthesis, efficient, fast, and stable membrane incorporation, selective binding of complementary DNA strands by Watson-Crick base pairing. Furthermore, for various purposes it would be desirable to achieve distinct patterns of specific lateral organisation of oligonucleotides in membranes. For example, by selecting appropriate lipid anchors enrichment of lipidated oligonucleotides in specific lipid domains should allow to create distinct functional compartments on a membrane surface. Local lateral recruitment of lipidated oligonucleotides in membranes may also provide a strategy to improve cellular uptake given the fact that endocytotic processes involve specific lipid domains. Previous attempts to lipidate nucleosides have resulted in an unselective double and triple lipidation, e. g. in esterification with fatty acids.. Some researchers have tried to circumvent this by introducing a cholesterol at the 3'-end of oligonucleotides^{[1;8]}.

Yet there remains a need in the art to provide for oligonucleotides that have been selectively and specifically lipidated and that can be used for a variety of purposes including but not limited to the incorporation into siRNA. Accordingly, the object of the present invention is to provide for oligonucleotides that are lipidated in a selective manner. Moreover it has been an object of the present invention to provide for nucleosides that can be easily synthesized using standard organic chemistry synthesis methods. It has also been an object of the present invention to provide for lipidated nucleotides and phosporamidates that can be subsequently used in oligonucleotide synthesis, thereby allowing the introduction of lipidated nucleotides in a directed and specific manner.

All these objects are solved by
an oligonucleotide having formula 1 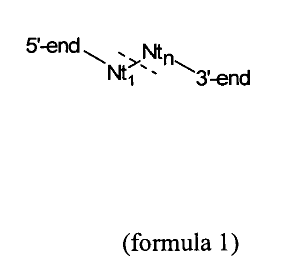 wherein
n denotes the total number of mononucleotides being present in said oligonucleotide, with n being ≥2,
the symbol " 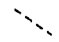 " signifies that there may be Nt₂, Nt₃ etc. between Nt₁ and Ntₙ, if n>2,
Nt₁, ..., Ntₙ are the same or different representing mononucleotides of the general formulae 2, 3, 4, or 5, 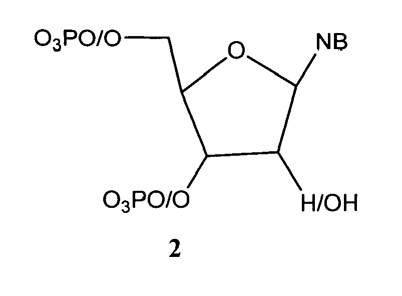 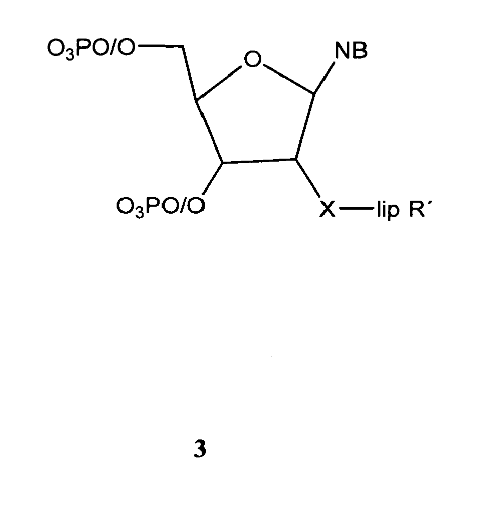 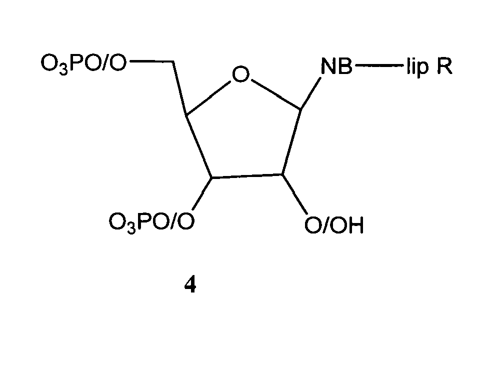 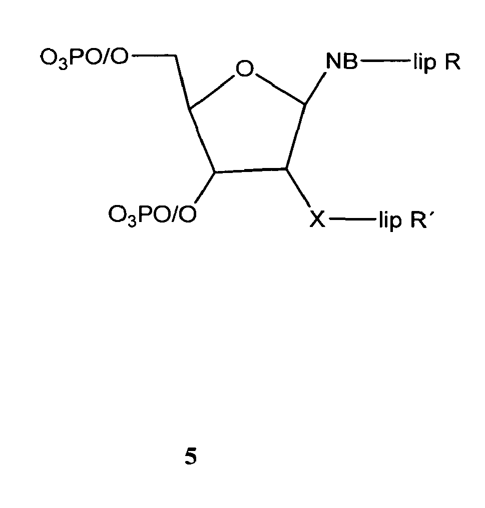 wherein at least one of Nt₁, ..., Ntₙ is a mononucleotide of formula 3, 4 or 5,
wherein X is O, N, S, phosphate or a single bond,
wherein lip R and lip R' are lipophilic moieties selected from the group comprising
fatty acyl groups having 6-30 C-atoms,
branched, unbranched, saturated or unsaturated open-chain or cyclic hydrocarbon groups having at least 10 carbon atoms,
branched, unbranched, saturated or unsaturated open-chain or cyclic hydrocarbon groups having at least 10 carbon atoms and one or several heteroatoms, like O, N or S,
terpenoid groups, such as sesquiterpenoids, diterpenoids, sesterterpenoids, triterpenoids, tetraterpenoids and polyterpenoids,
steroid groups,
lipid groups, such as diacylglycerol, wherein the two acyl chains are the same or different and have 6-30 C atoms, and phosphatidylcholines wherein the two acyl chains are the same or different and have 6-30 C-atoms, and phosphatidylethanolamines, wherein the two acyl chains are the same or different and have 6-30 C-atoms, and phosphatidyl serines, wherein the two acyl chains are the same or different and have 6-30 C-atoms, sphingolipids, and tocopherol groups,
wherein, in the case of lip R, said lipophilic moiety has a terminal C-C-triple bond, double bond or single bond and is linked to said NB via a terminal C of said triple bond, double bond or single bond, or wherein said lipophilic moiety is linked to said NB via a substituted or unsubstituted aromatic ring, preferably a benzene, a fluorene, an anthracene, more preferably a fluorescent aromatic ring, said ring forming part of lip R,
wherein, in the case of lip R', said lipophilic moiety is coupled directly or via -O- or via -S- or via -N- or via phosphate to the pentose ring,
wherein NB is a nucleobase se lected from the group comprising purine bases and pyrimidine bases, such as adenine, guanine, cytosine, uracil, thymidine, 7-methylguanine, 5-methylcytosine, hypoxanthine, 4-thiouracil, 5-hydroxymethylcytosine, N²-methylguanine, N⁶.Methyladenine, pseudouracil,
and wherein Nt₁ being the 5'-terminal mononucleotide has an OH- or phosphate group in the 5'-position and Ntₙ being the 3'-terminal mononucleotide has an OH- or phosphate group in the 3'-position, instead of the O indicated in these positions in formulae 2, 3, 4 and 5.

In one embodiment lip R or lip R' are -C≡C-C₁₂H₂₅, -C≡C-tocopheryl, -C≡C-C₁₆H₃₃, decynyl, undecynyl, dodecynyl, tridecynyl, tetradecynyl, hexadecynyl, octadecynyl, eisosynyl, or steroidylethynyl.

Preferably, lip R is -C≡C-C₁₂H₂₅, -C≡C-tocopheryl, -C≡C-C₁₆H₃₃, decynyl, undecynyl, dodecynyl, tridecynyl, tetradecynyl, hexadecynyl, octadecynyl, eisosynyl or steroidylethynyl.

In one embodiment n is selected from the range from 2 to 60.

Preferably the oligonucleotide according to the present invention comprises (n-1) mononucleotides according to formulae 3, 4 or 5.

In one embodiment, the oligonucleotide according to the present invention has n>2 and comprises at least 2, 3, 4, 5, ... or (n-2) mononucleotides according to formulae 3, 4 or 5.
In one embodiment, the oligonucleotide according to the present invention comprises n mononucleotides according to formulae 3, 4 or 5.

The objects of the present invention are also solved by a nucleic acid duplex comprising in one strand an oligonucleotide according to the present invention.

In a preferred embodiement, the nucleic acid duplex according to the present invention is siRNA.

The objects of the present invention are also solved by a nucleic acid vector comprising an oligonucleotide according to the present invention or a nucleic acid duplex according to the present invention.

The objects of the present invention are also solved by a lipid membrane comprising an oligonucleotide, a nucleic acid duplex, or a nucleic acid vector according to the present invention

Preferably, the lipid membrane has one or several lipid-disordered domains.

In one embodiment the lipid membrane has a lipid-ordered structure.

The objects of the present invention are also solved by a method of synthesis of an oligonucleotide according to the present invention, wherein at least one of Nt₁, .., Ntₙ is a mononucleotide of formula 4,
comprising the steps
- providing a mononucleoside of formula 10

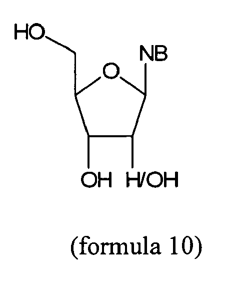

NB being as defined above , wherein the OH-groups, if present in the 2' position, the OH-groups in the 3' and/or 5'-positions may be and, if desired, are protected by protecting groups, such as silyl groups, acetonides, benzyl groups, including substituted benzyl groups, acyl groups, benzylidene, triarylmethyl
- providing
   either a compound of formula 6

   R₁-C≡C-Z (formula 6)

   R₁-C≡C representing lip R as defined in claim 1,
   Z being hydrogen, a metal or boron or a leaving group such as a halide or a triflate,
   or a compound of formula 7

   R₁-C=C-Z (formula 7)

   R₁-CH=CH representing lip R as defined in claim 1, Z being hydrogen, a metal or boron or a leaving group such as a halide or a triflate,
   or a compound of formula 8

   R₁-CH₂-CH₂-Z (formula 8)

   R₁-CH₂-CH₂ representing lip R as defined in claim 1,
   Z being hydrogen, a metal silica, phosphorus or boron or a leaving group such as a halide, p-toluolsulfonate or triflate,
   or a compound of formula 9

   Aryl-Z (formula 9)

   Aryl representing lip R as defined in claim 1, wherein lip R is a lipohilic moiety having a substituted or unsubstituted aromatic ring, preferably a benzene, a fluorene, an anthracene, more preferably a fluorescent aromatic ring, Z being hydrogen, a metal or boron or a leaving group such as halide or triflate,
- reacting said nucleoside having formula 10 as defined above with said compound of formula 6, 7, 8 or 9, in the presence of a metal catalyst, such as a Pd catalyst, a Ni catalyst or a Fe catalyst and/or a copper salt, to form a mononucleoside according to formula 11, 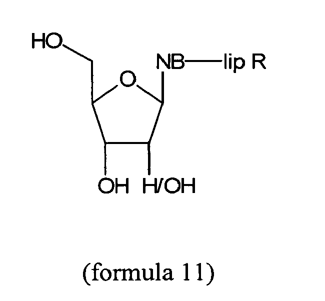 lip R and NB being as defined above and wherein the OH-groups, if present in the 2' position, the OH-groups in the 3' and/or 5'-positions may be and, if desired, are protected by protecting groups, such as silyl groups, acetonides, benzyl groups, including substituted benzyl groups, acyl groups, benzylidene, triarylmethyl,
- using said mononucleoside in solid or liquid phase oligonucleotide synthesis, to form an oligonucleotide according to the present invention, wherein at least one of Nt₁, .., Ntₙ is a mononucleotide of formula 4.

The objects of the present invention are also solved by a method of synthesis of an oligonucleotide according to the present invention, wherein at least one of Nt₁, .., Ntₙ is a mononucleotide of formula 3,
comprising the steps
- providing a mononucleoside of formula 14 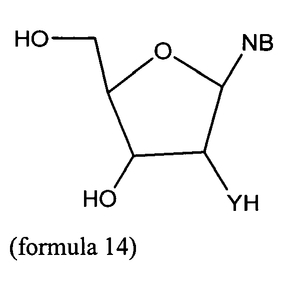 Y being one of O, NH, NAlkyl, S
   NB being as defined above, wherein the OH-groups in the 3' and/or 5'-positions may be and, if desired, are protected by protecting groups, such as silyl groups, benzyl groups, including substituted benzyl groups, acyl groups, benzylidene, triarylmethyl
- providing an acylation reagent having lip R' attached or an alkylating reagent having lip R' attached, said acylation reagent having the formula lip R'-COZ', Z' being a leaving group, such as OH, halogen, acyloxy, imidazole, said alkylating reagent having the formula Z-lip R', Z being a leaving group such as OH, halogen, a sulfonate, such as trifluoromethanesulfonate, tosylate, benzoylsulfonate, or providing a heterocumulene of the formula X=C=N-lip R' with X = O, S, NAlkyl, NAryl, or providing a phosphorylating reagent having lip R' attached, said heterocumulene being preferably an isocyanate, isothiocyanate or a carbodiimide, and
- reacting said mononucleoside of formula 14 with said acylation reagent, alkylating reagent, heterocumulene, or phosphorylating reagent, in the presence of a metal catalyst, such as a Pd catalyst, a Fe catalyst or a Ni catalyst and/or a copper salt, to form a mononucleoside according to formula 12 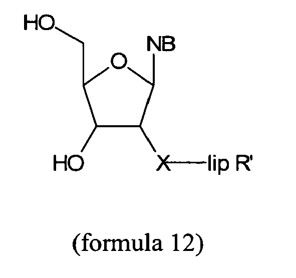
- using said mononucleoside in solid or liquid phase oligonucleotide synthesis, to form an oligonucleotide according to the present invention, wherein at least one of Nt₁, .., Ntₙ is a mononucleotide of formula 3.

The objects of the present invention are also solved by a method of synthesis of an oligonucleotide according to the present invention, wherein at least one of Nt₁, .., Ntₙ is a mononucleotide of formula 5 comprising the steps
- providing a mononucleoside of formula 10 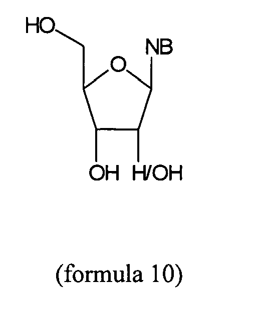 NB being as defined in claim 1,
- attaching, in any order, a lip R moiety at the nucleobase by a reaction for attaching such moiety at the nucleobase as defined above, and a lip R' moiety at the 2'-position of the pentose by a reaction for attaching such moiety at the 2' position as defined above, to form a mononucleoside according to formula 13 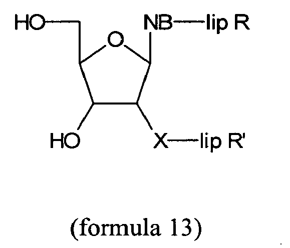 wherein the OH-groups in the 3' and/or 5'-positions may be and, if desired, are protected by protecting groups such as silyl groups, benzyl groups, acyl groups, benzylidene, triarylmethyl,
- using said mononucleoside in solid or liquid phase oligonucleotide synthesis to form an oligonucleotide according to the present invention, wherein at least one of Nt₁, .., Ntₙ is a mononucleotide of formula 5.

It is clear to someone skilled in the art that attachment of a lip R' moiety at the mononucleoside of formula 10 in the 2'-position occurs via an intermediate mononucleoside of formula 14 as described further above, having a OH, NH₂, SH, NHAlkyl or other suitable functional group in the 2' position the introduction of which functionality can be easily performed by standard chemical reactions.

Solid phase oligonucleotide synthesis is known to someone skilled in the art and is further described in detail below.

The objects of the present invention are also solved by the use of an oligonucleotide according to the present invention for incorporation into a nucleic acid duplex, preferably small interfering RNA (siRNA).

The objects of the present invention are also solved by the use of an oligonucleotide according to the present invention and/or a nucleic acid duplex according to the present invention, and/or a nucleic acid vector according to the present invention for facilitating the uptake of nucleic acids into cells.

The objects of the present invention are also solved by the use of an oligonucleotide according to the present invention and/or a nucleic acid duplex according to the present invention, and/or a nucleic acid vector according to the present invention for structuring a lipid membrane, preferably a lipid bilayer membrane, and/or for establishing/immobilizing/arranging a chemically reactive complex in a lipid membranes ("nanoreactor"), preferably different enzymes involved in the same reaction (cascade) which are arranged in close neighborhood on a lipid membrane, and/or for structuring surfaces of nucleic acid chips having lipophilic surfaces, and/or for establishing molecular receptors in lipid membranes.

The objects of the present invention are also solved by a method of synthesis of an oligonucleotide ("solid or liquid phase oligonucleotide synthesis") according to the present invention, comprising the steps:
1. step: protection of 5'-hydroxy group of a nucleoside of formula **2, 3, 4 or 5** by a protective group
2. step: transformation of the 3'-hydroxy group into a phosphoramidite or phosphate
3. step: coupling of the 5 '-protected 3'-phosphoramidite or phosphate with the 5'-position of another nucleotide or oligonucleotide, which can be linked to a solid support.to give the coupling product
4. capping of unreacted nucleotide or oligonucleotide
5. if the newly formed bridge between the last two nucleotides represents a phosphite, this phosphite is transformed to a phosphate by oxidation
6. step: deprotection of the 5'-hydroxy group of the coupling product
7. repetition of steps 1-6
8. deprotection of the nucleobases and the 5'-hydroxy group and cleavage of the oligonucleotide from the solid support, as far as the synthesis is carried out on solid support.

A typical procedure for step 1 is as follows:
Thymidine is dissolved in dry pyridine (50- 100 ml per 10 mmol nucleoside). 4-Dimethylaminopyridine (0.05 equivalents), triethylamine (1.4 equivalents) and 4,4'-dimethoxytrityl chloride (1.2 equivalents) are added. After reaction has gone to completion one equal volume of water is added and the mixture is extracted with diethyl ether (2 times with 500 ml). Concentration of the organic layer with a rotatory evaporator gives a white foam, which can by purified by recrystallisation from benzene or by column chromatography.

A typical procedure for step 2 is as follows:
The 5'-DMTr-protected nucleoside (1.09 mmol) was dissolved in dry dichloromethane. Tetrazole (1.63 mmol) dissolved in acetonitrile was added and then bis(N,N-diisopropylamino)-2cyanoethoxyphosphine (1.42 mmol).The mixture was stirred in an inert atmosphere for 5 h. The mixtue was concentrated under vacuum, diluted with ethyl acetate, washed with 5% aqueous NaHCO₃, brine. After drying the organic layer was evaporated and co-evaporated with toluene and the residue was chromatographed on silica gel.

Steps 3 to 8 can be conveniently be carried out in a DNA synthesizer.

It should be noted that in oligonucleotides having at least one mononucleotide of formula 4, lip R is not 1-pyrenyl, pyren-1-ylethynyl, decyn-1-yl, dodecyn-1-yl, 7,8,9,11,12,13,14,15,16,17-decahydro-17-hydroxy-3-methoxy-13-methyl-6*H*-cyclopenta[*a*]phenanthrene-17-ethynyl, 7,8,9,11,12,13,14,15,16,17-decahydro-3, 17-dihydroxy-13-methyl-6*H-*cyclopenta[*a*]phenanthrene-17-ethynyl

As used herein, the term "hydrocarbon group" is meant to refer to a group having H- and C-atoms in their structure and having at least 10 carbon atoms. Typical examples of hydrocarbon groups in accordance with the present invention are decyl, undecyl, dodecyl, tridecyl, tetradecyl, hexadecyl, octadecyl, eicosyl, myricyl decynyl, undecynyl, dodecynyl, tridecynyl, tetradecynyl, hexadecynyl, octadecynyl, eisosynyl... decenyl, undecenyl, dodecenyl, tridecenyl, tetradecenyl, hexadecenyl, octadecenyl, eisosenyl...

The term "lipophilic moiety", as used herein is meant to refer to a moiety which has very little, if any solubility in water or aqueous solutions. A lipophilic moiety provides the molecule to which it is attached with the capability of interacting with other lipids and/or incorporation into lipid membranes and/or the formation of micelles.

The terms "saturated" and "unsaturated", as used herein, when referring to hydrocarbon groups refer to the absence and presence, respectively, of one or several double bonds or triple bonds or an aromatic system in said hydrocarbon group.

The term "terpenoid group", as used herein, refers to oligomers of isoprene-units. Depending on the number of isoprene units, one can distinguish monoterpenoids (C10), sesquiterpenoids (C15), diterpenoids (C20), sesterterpenoids (C25), triterpenoids (C30), tetraterpenoids (C40) and polyterpenoids (>C40). For example the terpenoid group can be geranyl, farnesyl or phytyl.

The term "steroid", as used herein, refers to compounds having a characteristic tetracyclic carbon structure of the perhydrogenated or partially hydrogenated cyclo-penta[a]phenanthrene. Typical examples of steroids are cholesterol, stigmasterol, androgens, estrogens, gestagens, corticosteroids, strophanthines, etc.

The term "tocopherol", as used herein refers to croman-6-ol being substituted in 2-position with a 4,8,12-trimethyltridecyl-rest.

The term "nucleobase", as used herein is meant to refer to unmodified and modified purine bases and pyrimidine bases. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine(5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine und 8-azaadenine, 7-deazaguanine and 7-deazaadenine and 3-deazaguanine and 3-deazaadenine. Further modified nucleobases include tricyclic pyrimidines such as phenoxazine cytidine (1H-pyrimido[5,4-b][1,4]benzoxacin-2(3H)-one), phenothiazine cytidine (1H-pyrimido[5,4-b][1,4]benzothiazine-2(3H)-one), G-clamps such as substituted phenoxazine cytidine (e.g. 9-(2-aminoethoxy)-H-pyrimido[5,4-b][1,4]benzoxazine-2(3H)-one, carbazole cytidine(2H-pyrimido[4,5-b]indol-2-one), pyridoindole cytidine(H-pyrido[3'2':4,5]pyrrolo(2,3-d)pyrimidine-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in US-patent no. 3,687,808, those disclosed in The Concise Encyclopaedia of Polymer Science and Engineering, pages 858-859, Kroschwitz, J.I. Ed., John Wiley and Sons, 1990, those disclosed by Englisch et al., Angewandte Chemie, International Edition 1991, 30, 613, and those disclosed by Sanghvi, Y.S., Chapter 15, Antisense Research and Applications, pages 289-302, Crooke, S.T. and Lebleu, B. Ed., CRC Press 1993. Certain of these nucleobases are particularly useful for increasing the binding affinity of the nucleotides/oligonucleotides of the present invention in nucleic acid duplices. These include 5-substituted pyrimidines, 6-azapyrimidines and N-2, N-6 and O-6 substituted purines, including 2-aminopropyl adenine, 5-propynyl uracil and 5-propynyl cytosine. 5-methylcytosine substitutions have been shown to increase nucleic acid duplex stability by 0.6-1.2°C (Sanghvi, Y.S., Crooke, S.T. and Lebleu, B., Eds. Antisense Research and Applications, CRC Press, Boca Raton, 1993, pp. 276-278).

The term "siRNA", as used herein is meant to refer to short interfering RNA which is doublestranded RNA having symmetrical overhangs (2-3 nucleotides) at the 3'-end, a phosphate group at the 5'-end and a free hydroxy group at the 3'-end. Using siRNA it is possible to temporarily switch off specific genes in that the siRNA causes the degradation of the corresponding mRNA such that no translation occurs anymore.

Methods of synthesis of oligonucleotides are known to someone skilled in the art and can for example be performed using standard phosphoramidite chemistry, as for example described in . M. J. Gait, Oligonucleotie synthesis: a practical approach, IRL Press, Oxford, Washington DC, 1984. S. Müller, J. Wolf, S. A. Ivanov, Current Organic Synthesis 2004, 1, 293-307.

The term "nanoreactor" as used herein is meant to refer to a chemically reactive complex which has a desired functionality, such as for example catalysis of a specific reaction For example, enzymes taking part in a series of reactions, e.g. in a biochemical pathway, may be immobilised in close proximity to each other on/in a lipid membrane thus forming a nanoreactor. The prefix "nano" in this term refers to the fact that such reactor usually is not bigger than 1 µm.

Here the present inventors describe synthesis and application of lipidated single stranded nucleic acid molecules that stably insert into lipid membranes, display the oligonucleotide on the vesicular surface, bind complementary nucleic acid strands, e.g. DNA strands by formation of Watson Crick base pairs, and preferentially sequester into lipid-disordered or other membrane domains or not. They further describe the synthesis und application of lipidated double stranded nucleic acid molecules that stably insert into lipid membranes, display the oligonucleotide on the vesicular surface and preferentially sequester into lipid-disordered or other membrane domains or not.

Using the approach in accordance with the present invention, it is possible to produce nucleotides that are lipidated by any desired lipophilic moiety in the nucleobase position and/or the 2'-position. The structure and properties of a lipid moiety are essential for modulating the membrane affinity, for the specific localization in lipid domains and for lateral diffusion. The lipidated nucleotides thus produced can subsequently be used for oligonucleotides syntheses and can therefore be introduced in any position in the growing oligonucleotide chain. In doing this, the qualities of the oligonucleotides thus produced can be systematically influenced and manipulated in a directed manner. Furthermore, the nucleotides/oligonucleotides according to the present invention can be influenced in terms of the number, position (nucleobase or 2'-position) and the type of lipophilic groups that are attached. The oligonucleotides can be varied in terms of the number of incorporated lipidated nucleotides, their type of lipidation, their position etc., thus influencing the overall qualities of the oligonucleotide thus produced. Moreover, the oligonucleotides according to the present invention may be incorporated into lipid membranes, for example lipid bilayer membranes as they are found in cell membranes, and, in these membranes, the oligonucleotides may be sorted and enriched, depending on the lipophilic nature of their nucleotides, into specific lipid domains. Furthermore, the oligonucleotides according to the present invention may be hybridised to complementary oligonucleotides, whilst being in solution or whilst being incorporated in a lipid membrane. Furthermore, the nucleotides/oligonucleotides according to the present invention are useful for facilitating the uptake and transfer of siRNA or other short nucleic acids into cells, due to the lipophilic moieties attached to them.

The present invention therefore has opened up a feasible way of manipulating nucleic acid building blocks and their interactions with hydrophobic surfaces, such as lipid membranes, in a manner hitherto unknown.

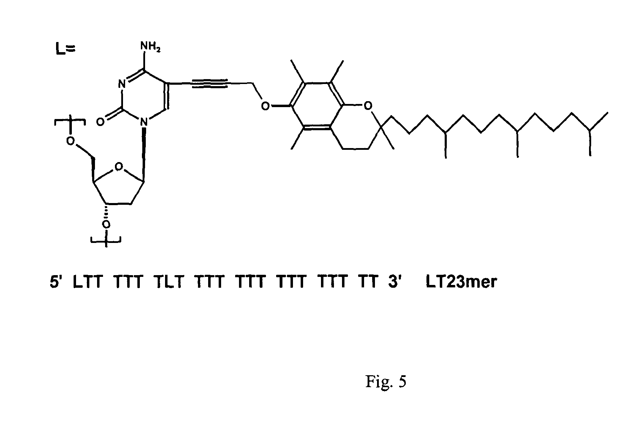

One example of an oligonucleotide according to the present invention is the lipophilic oligonucleotide **L23Tmer.** The lipophilic oligonucleotide **L23Tmer** is a 25mer consisting of 23 thymidine units and two cytosine nucleotides X with a α-tocopherol unit as lipohilic anchor (see also figure 5). These lipophilic cytosines are found in position 1 and 8. **L23Tmer** could be synthesized by a DNA synthesizer applying phosphoramidite methodology. The lipophilic tocopherylpropynylcytosine was obtained by Sonogashira-coupling and transformed to the 5'-DMTr-protected 3'-diisopropylaminocyanethylphosphoramidite by conventional methods. The Sonogashira coupling is described in greater detail further below. This methodology is very flexible because lipophilic nucleotides can be introduced in any position of a oligonucleotide.

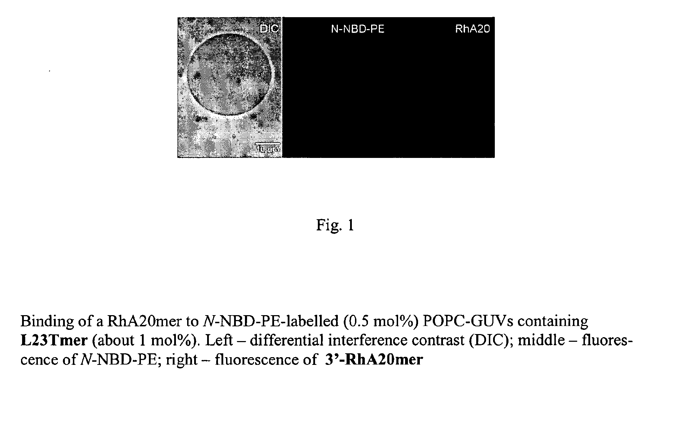

In the following, reference is made to the figures, wherein
figure 1 shows the binding of a RhA20mer to *N*-NBD-PE-labelled (0.5 mol%) POPC-GUVs containing **L23Tmer** (about 1 mol%). Left - differential interference contrast (DIC); middle - *N-*NBD-PE; right - **3'-RhA20mer,**

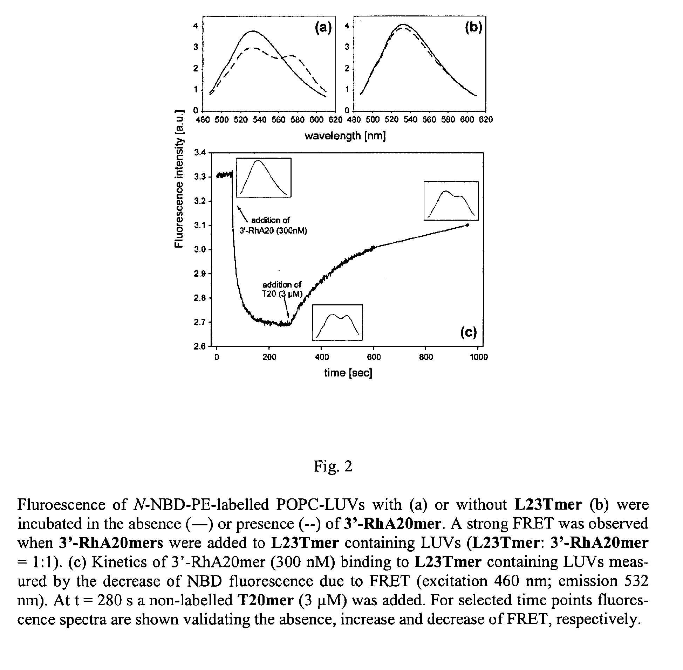

figure 2 shows fluroescence of *N*-NBD-PE-labelled POPC-LUVs with (a) or without **L23Tmer** (b) were incubated in the absence (-) or presence (--) of **3'-RhA20mer.** A strong FRET was observed when **3'-RhA20mers** were added to **L23Tmer** containing LUVs **(L23Tmer: 3'-RhA20mer** = 1:1). (c) Kinetics of 3'-RhA20mer (300 nM) binding to **L23Tmer** containing LUVs measured by the decrease of NBD fluorescence due to FRET (excitation 460 nm; emission 532 nm). At t = 280 s a non-labelled **T20mer** (3 µM) was added. At selected time point the whole fluorescence spectra are shown in (a) and (b) validating the absence, increase and decrease of FRET, respectively,

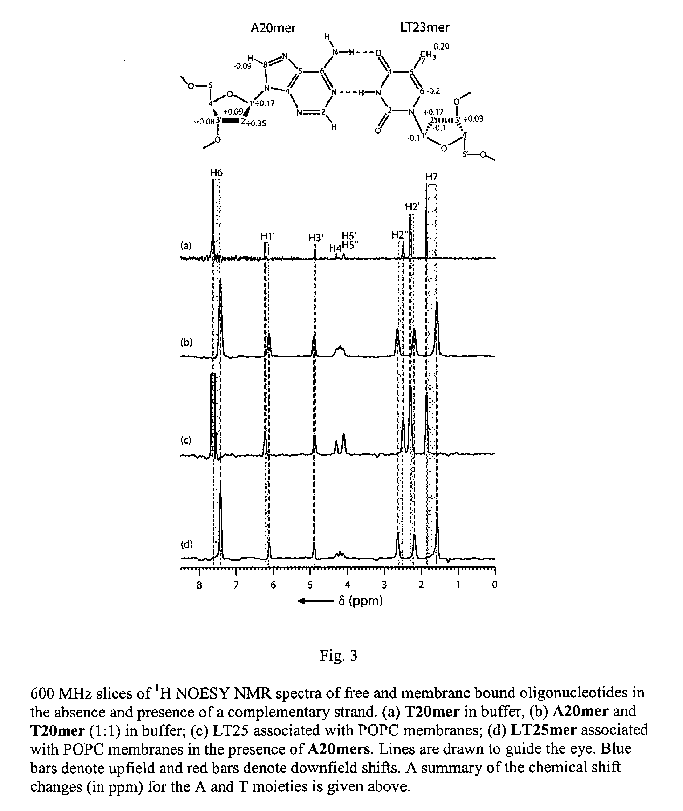

figure 3 shows 600 MHz slices of ¹H NOESY NMR spectra of free and membrane bound oligonucleotides in the absence and presence of a complementary strand. (a) **T20mer** in buffer, (b) **A20mer** and **T20mer** (1:1) in buffer; (c) LT25 associated with POPC membranes; (d) **LT25mer** associated with POPC membranes in the presence of **A20mers**. L ines are drawn to guide the eye. Blue bars denote upfield and red bars denote downfield shifts. A summary of the chemical shift changes (in ppm) for the A and T moieties is given above,

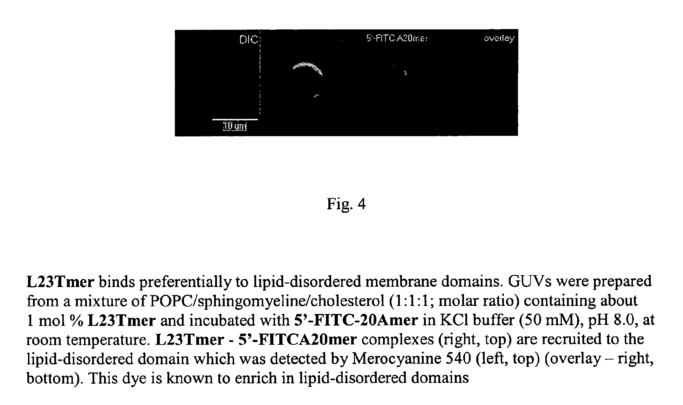

figure 4 shows that **L23Tmer** binds preferentially to lipid-disordered membrane domains. Giant unilamellar resides (GUVs) were prepared from a mixture of POPC/sphingomyeline/cholesterol (1:1:1; molar ratio) containing about 1 mol % **L23Tmer** and incubated with **5'-FITC-20Amer** in KCl buffer (50 mM), pH 8.0, at room temperature. **L23Tmer - 5'-FITCA20mer** complexes (right, top) are recruited to the lipid-disordered domain which was detected by Merocyanine 540 (left, top) (overlay - right, bottom). This dye is known to enrich in lipid-disordered domains,
figure 5 shows the structure of **L23Tmer** as an example of an oligonucleotide according to the present invention,

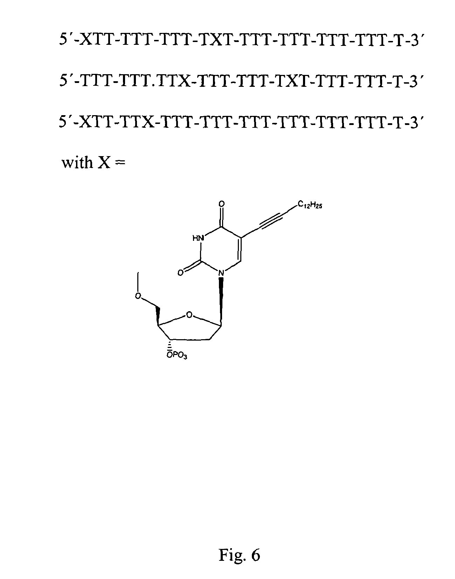

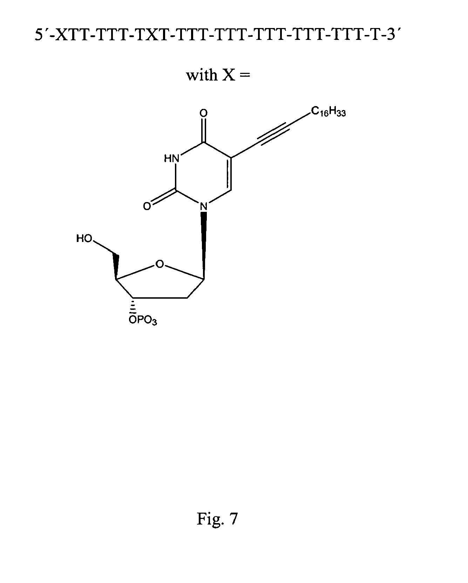

figures 6 and 7 show further nucleotides/oligonucleotides synthesized in accordance with the present invention, and

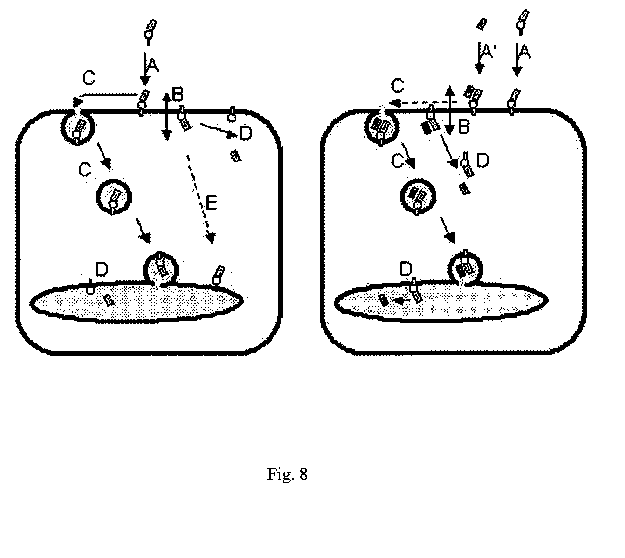

figure 8 shows a schematic representation of the intracellular uptake of nucleic acid building blocks, wherein A is the incorporation in the outer layer of the plasma membrane (A' = noncovalent binding of a nucleic acid strand); B = transversal movement across the membrane; C = endocytotic uptake; D = (enzymatic) cleavage of a covalent bond (left) or dissociation of a noncovalent bond (right); E = intracellular transport/diffusion.

Furthermore reference is made to the following examples which are given to illustrate, not to limit the present invention.

### Examples

### a) synthesis of nucleotide/oligonucleotides

The compounds shown in figures 5 - 7 were successfully synthesised using a Sonogashira coupling reaction of 5-iodouridin or 5-iodo-2'-desoxyuridine and the corresponding alkyne in the presence of a Pd catalyst together with CuI and iPr₂EtN in DMF. Additional examples and reaction details are given below in section g).

### b) membrane affinity and oligonucleotide binding by fluorescence microscopy

After synthesis the membrane affinity of the molecule and the ability to bind a complementary oligo(A) DNA strand was tested. First, fluorescence microscopy was used to visualize the association of **L23Tmer** with giant unilamellar vesicles (GUV). GUVs were fluorescently labelled with the phospholipid analogue *N*-NBD-PE (0.5 mol%) and contained 1 mol% **L23Tmer**. To demonstrate binding of the complementary DNA strand, a 20mer adenine oligonucelotide (**3'-Rh20Amer**) tagged on its 3' terminus with the acceptor fluorophore rhodamine (Rh) was added. GUV images are shown in Fig. 1. In the presence of **L23Tmer** the GUV membranes could be identified by the fluorescence of **3'-Rh20Amers.** Fluorescence was homogenous all over the membrane indicating that membrane bound **L23Tmer** is homogenously distributed within the membrane. GUVs did not become labelled by a rhodamine tagged **20Tmer.** Furthermore, in the absence of **L23Tmer** no labelling of GUVs with **3'-Rh20Amers** was observed (image not shown).

### c) oligonucleotide binding by fluorescence spectroscopy

Second, to assess whether membrane bound **L23Tmer** binds complementary adenine oligonculeotides the present inventors employed a fluorescence resonance energy transfer (FRET) assay. LUVs containing either POPC and **L23Tmer** or only POPC (control) were prepared in the presence of *N*-NBD-PE (0.5 mol%). The fluorescent NBD moiety which serves as a donor is covalently attached to the lipid head group. These LUVs were incubated with the **3'-Rh20Amer** with Rh being the acceptor. Binding of this oligonucelotide to the membrane surface can be followed by FRET. The present inventors measured a strong FRET when LUVs containing **L23Tmer** were incubated with **3'-Rh20Amer** (Fig. 2a). FRET was not observed for control LUVs containing only POPC (neither **L23Tmer** nor DOTAP) (Fig. 2b).
Binding of 3'-RhA20 to LUVs with membrane bound **L23Tmer** is rapid. The half time of binding was about 20 s at 20°C (Fig. 2c). To demonstrate that binding of **3'-RhA20mer** is reversible the present inventors added a (non-labelled) **T20mer** in 10 fold excess with respect to **L23Tmer** As can be seen the present inventors observed a decrease of FRET due to the competition of the **T20mer** for **3'-RhA20mer**.

### d) structure of oligonucleotide duplex by NMR spectroscopy

Third, the present inventors investigated the structure of the membrane associated complex of **L23Tmer** and poly(A) by NMR spectroscopy. As a control, solution NMR studies of stoichiometric mixtures of **A20mer** and **T20mer** were carried out. It is known that base pair formation and stacking leads to characteristic changes of the NMR chemical shifts.^{[10]} In Fig. 3, slices of ¹H NOESY spectra are shown. In contrast to the ¹D spectra that also contain large signals from the lipid membrane, the NOESY slices only show the signals of the base and the sugar moiety. Fig. 3a shows the ¹H NMR spectrum of **T20mer.** In the presence of the complementary **A20mer** strand, all resonance lines are shifted in a specific manner (b). The **L23Tmer** bound to POPC LUVs (c) shows exactly identical ¹H chemical shifts as isolated **T20mer**. After addition of the complementary **A20mer**, identical relative shifts compared to the **A20mer-T20mer** double helix are observed (d). The chemical shift changes of **A 20mer** after binding to either **T20mer** or **L23Tmer** are also identical (not shown). This strongly suggests that very similar structures are formed and the membrane proximity does not interfere with the formation of the DNA double helix.

### e) lipid-disordered and -ordered domains

It has been shown that GUVs prepared from a lipid mixture of POPC, cholesterol and sphingomyeline (1:1:1) form lipid-disordered and lipid-order domains.^{[11-13]} Domains can be visualized by fluorescence microscopy employing fluorescent lipid analogues which preferentially enrich in one of the two domains. For example, the lipophilic fluorophore Merocyanine 540 incorporates spontaneously into lipid membranes and is known to accumulate into the lipid-disordered domain.^{[14]} The present inventors have used GUVs of such a lipid mixture (1:1:1 (molar ratio)) containing **L23Tmer** (about 1 mol%) and labelled with Merocyanine 540. Subsequently, complementary oligonucleotides **(5'-FITC-20Amer)** were added. In contrast to the homogeneous distribution in pure POPC GUVs (see Fig. 1), the present inventors observed that the **L23Tmer** with the complementary strand was essentially recruited to the lipid-disordered domain identified by Merocyanine 540 staining (Fig. 4). A preferential recruitment of a lipid disordered phase can be rationalized based on the structure of the lipid anchor. The bulky α-tocopherol anchor could pack more easily into a lipid-disordered phase.

The present inventors have shown a simple strategy to synthesize lipophilic nucleosides and nucleotides that can be used to produce large quantities of lipid anchored oligonucleotides with variable length and primary structure. These molecules insert stably into lipid membranes and expose the ssDNA to the aqueous phase. Complementary strands are recognized and self-assemble into double stranded DNA with Watson-Crick base pairing. This property could be used to tether ssDNA and dsDNA to specific domains of heterogeneous lipid surfaces for the first time but can also be used to tether ssDNA or dsDNA to homogenous lipid surfaces. The present inventors' results imply that molecules, vesicles, drugs, or biologically active RNA can be associated with membrane or cellular surfaces at will. Given the fact that lipid domains play an important role in endocytosis,^{[15]} specific enrichment of lipophilic oligonucleotides to those domains could be explored to enhance cellular uptake. Thus, nanostructures may be built and various cell biological or medical applications can be foreseen.

### f) further experimental details

1-Palmitoyl-2-oleoyl-*sn*-glycerophosphocholine (POPC), sphingomyeline (SM), and the fluorescent lipid *N*-(7-nitro-2,1,3-benzoxadiazol-4-yl)-1,2-dipalmitoyl-*sn*-glycero-3-phosphatidyl-ethanolamine (*N*-NBD-PE) were obtained from Avanti Polar Lipids (Birmingham, AL, USA). Cholesterol and Merocyanine (M540) were from Sigma (Deisenhof, Germany). DNA-oligonucleotides without or with a covalently attached fluorophores (rhodamine) were synthesized by BioTez (Berlin-Buch, Germany). All solutions used were buffered with 10 mM HEPES, pH 8. The preparation of unilamellar vesicles was performed in sucrose solution (80 to 250 mM). For washing and dilution of the vesicles and preparation of multilamellar vesicles buffered KCL, KCL-glucose or glucose solutions of equal osmolarity were used (see below).
Multilamellar vesicles (MLVs) were formed by resuspension of a lipid film by manual swirling of the glass flask. For preparation of large unilamellar vesicles, the MLV suspension was subjected to five freeze-thaw-cycles and extruded through a polycarbonate filter (Nucleopore GmbH, Tübingen, Germany) of 100 nm diameter using an extruder (Extruder, Lipex Biomembranes Inc., Vancouver, Canada).^{[16]} For membrane incorporation, L23Tmers were added to an aliquot of the LUVs and the suspension was again subjected to five freeze-thaw-cycles. Subsequently, unbound L23Tmers and any micelles remained were removed by ultracentrifugation.
Giant unilamellar vesicles (GUVs) were generated at room temperature (RT) by the electro formation technique in a chamber made of indium-tin-oxide coated glass slides.^{[17;18]} Fluorescence spectra and kinetics were recorded using an Aminco Bowman spectrometer series 2 (SLM-Aminco, Rochester, NY). *N*-NBD-PE was excited at 460 nm (slit width 4 nm) and fluorescence spectra were recorded between 470 and 610 nm with a scan rate of 1 nm/s whereas for kinetic measurements the emission wavelength was set to 532 nm (slit width 4 nm). GUVs were examined with an Olympus IX-81 inverted fluorescence microscope (Olympus, Hamburg, Germany) equipped with a cooled CCD camera (SPOT slider, Visitron Systems, Puchheim, Germany). Images were acquired using 100x Plan-APO oil immersion objective with the appropriate differential interference contrast (DIC) optics and fluorescence filter sets: BP 470-490, FT 505, and BP 510-550 (NBD, FITC); BP 530-550, FT 5 80, and LP 590 (rhodamine, merocyanine).
For NMR experiments, aliquots of **L23Tmer** were added to extruded 100 nm POPC vesicle suspensions (20 mM in D₂O buffer 10 100 7.4) at a **L25mer** to POPC molar ratio of 1:100. NMR experiments were carried out on a Bruker DRX600 or Avance 700 NMR spectrometer at a resonance frequency of 600.13 MHz or 700.13 MHz, respectively, at 30°C. Spectra were acquired at a spectral width of 7 kHz with a 90°pulse length of 10.3 µs. For phase sensitive NOESY experiments (mixing time 300 ms) 480 complex data points were collected in the t₁ dimensions with 32 or 64 scans per increment at a 4 s relaxation delay. Subsequently, aliquots of **A20mer** was added at a 1:1. molar ratio with respect to **L25mer** and NMR spectra were collected again.

### g) additional examples of reactions and reaction products

The following two compounds were synthesized starting from 5-iodouridine. The steroide containg alkyne was introduced by Sonogashira coupling. Then, the 3'and 5'hydroxy group were protected by di(t-butyl)silylen group and the 2'OH group was alkylated with 1,9-dibromononane. Finally the 2'-9-bromononyloxy group was extended by reaction with the nitroxylhexyl alcohol under basic conditions.

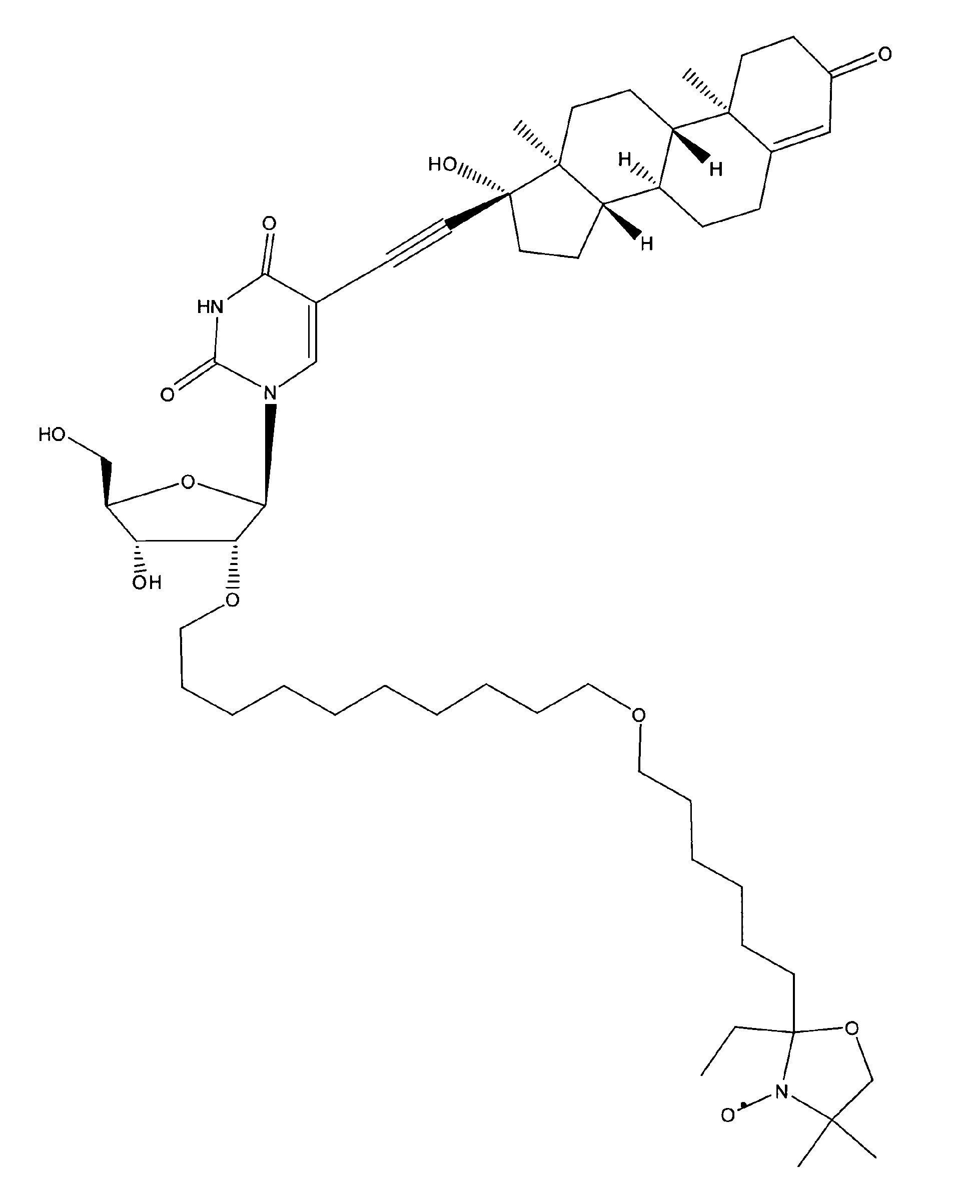

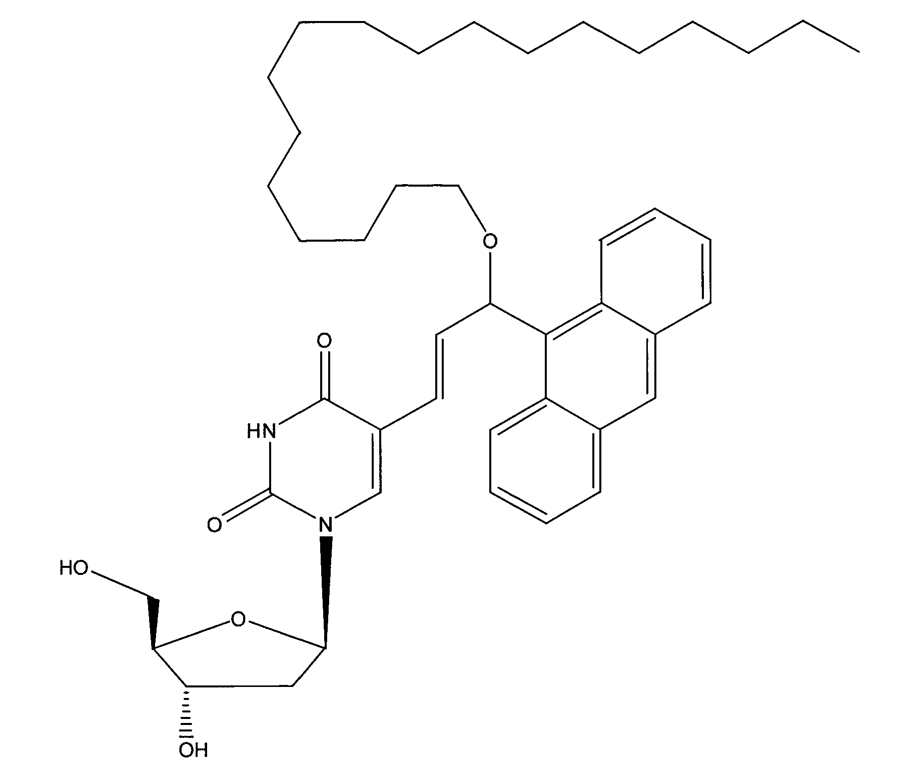

Using the above approach 5-ioduridine and 5-iodo-2'-desoxyuridine were subjected to a Sonogashira coupling in an unprotected manner using a number of different catalysts.

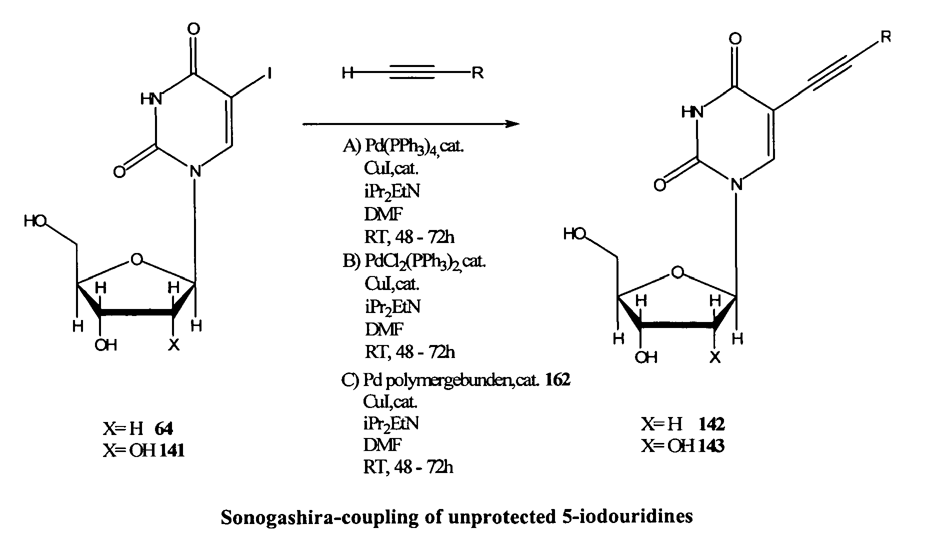

In most cases, the desired products could be isolated in very good yields. Thus the Sonogashira coupling is very tolerant with respect to the type of lipophilic group being introduced (see also table 1).

**table 1**

| Nr | X | C≡CR | Yield (%) |
|---|---|---|---|
| **142 a** | H | C≡C-*n*-C₁₆H₃₃ | 28 |
| **142 b** | H | C≡C-*n*-C₁₂H₂₅ | 99 |
| **142 c** | H | 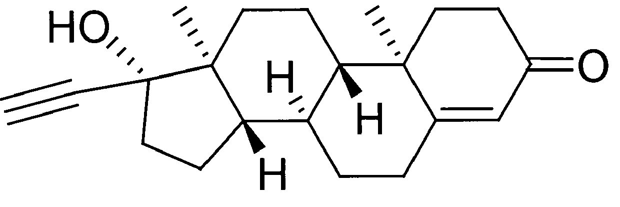 | 76 |
| **143 a** | OH | C≡C-*n*-C₁₆H₃₃ | 99 |
| **143 b** | OH | 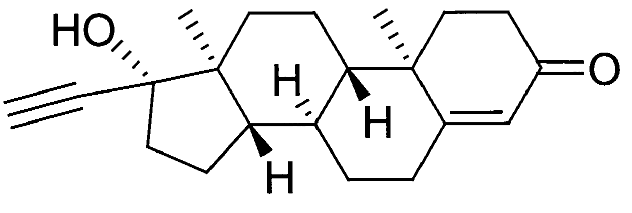 | 99 46 |
| **143 c** | OH | 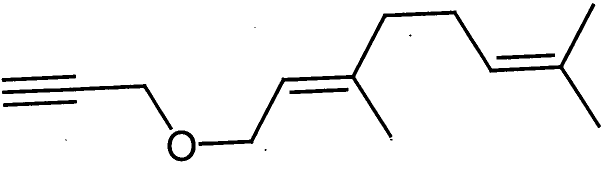 | 81 |
| **143 d** | OH | 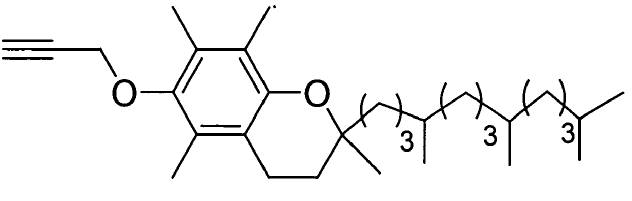 | 97 |
| **143 e** | OH | 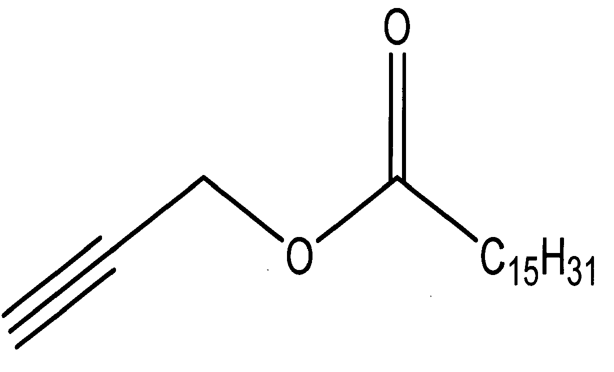 | 9 |

In a series of experiments, the iodouridines were protected prior to the coupling reaction to achieve a better solubility and easy work-up and to possibly make use of the protecting groups in subsequent reactions (see also table 2).

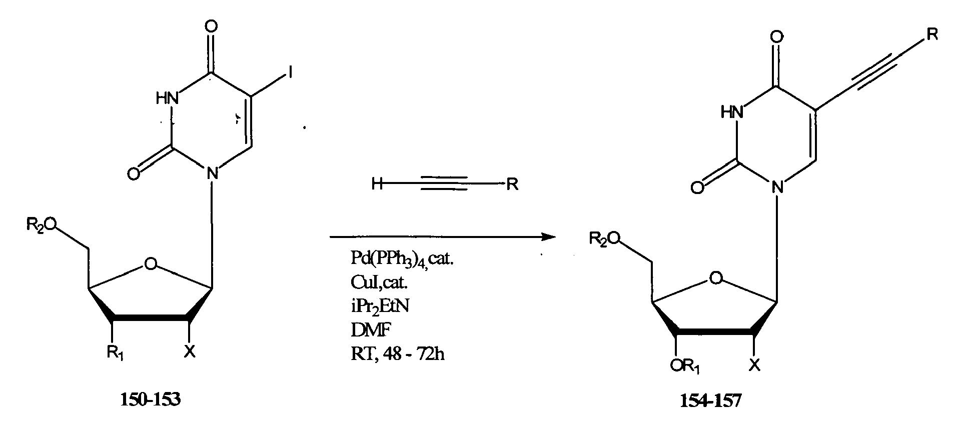

**table 2**

| Nr | R₁ | R₂ | X | C≡CR | yield (%) |
|---|---|---|---|---|---|
| **154 a** | H | DMTr | H | 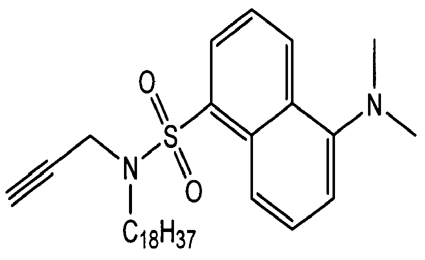 | 29 |
| **154 b^{p}** | H | DMTr | H | C≡C-*n*-C₁₆H₃₃ | 69 |
| **155°** | Bn | Bn | OBn | C≡C-*n*-C₁₆H₃₃ | 50 |
| **156°** | 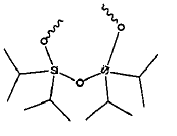 | | H | 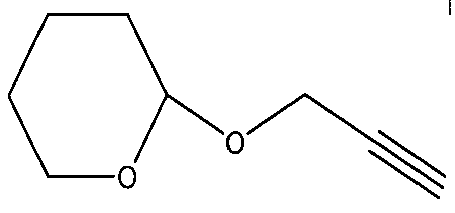 | 49 |
| **157 a°** | Ac | Ac | Ac | 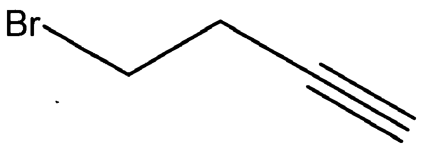 | 72 |
| **157 b°** | Ac | Ac | Ac | 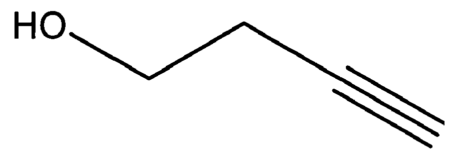 | 80 |

Moreover different adenosine derivatives having lipophilic moieties in the 8-position were produced:

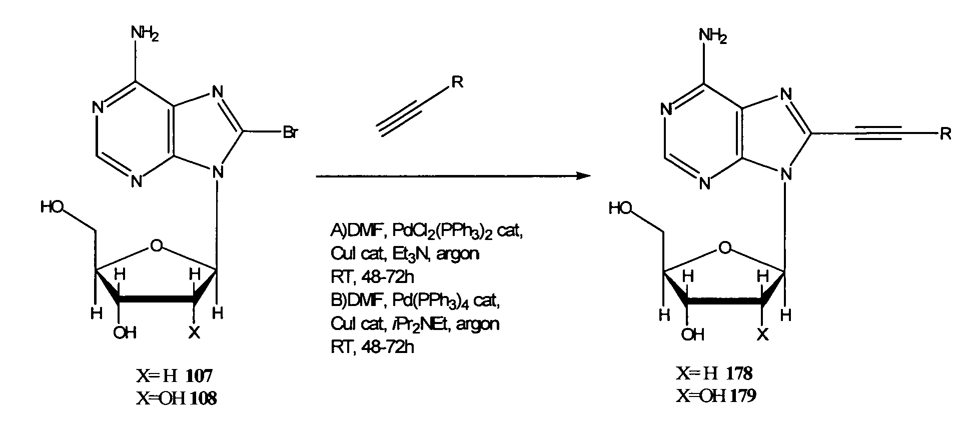

**table 2**

| Nr | X | C≡CR | yield (%) |
|---|---|---|---|
| **178 a** | OH | C≡C-*n*-C₁₂H₂₅ | 23 |
| **178 b** | OH | C≡C-*n*-C₁₄H₂₉ | 80 |
| **178 c** | OH | C≡C-*n*-C₁₆H₃₃ | 82 |
| **178 d** | OH | C≡C-*n*-C₁₈H₃₇ | 97 |
| **178 e** | OH | 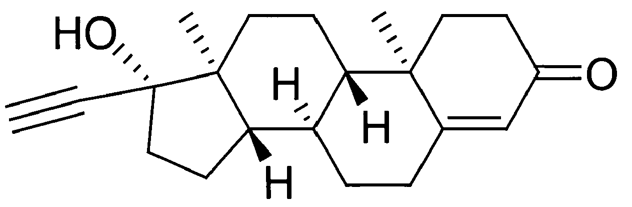 | 76 31 |
| **179 a** | H | C≡C-*n*-C₁₂H₂₅ | 73 |
| **179 b** | H | C≡C-*n*-C₁₄H₂₉ | 56 |
| **179 c** | H | C≡C-*n*-C₁₆H₃₃ | 83 |

Moreover the Sonogashira coupling reaction was used to synthesise three extremely lipophilic cytidine derivatives 187 c, d and e:

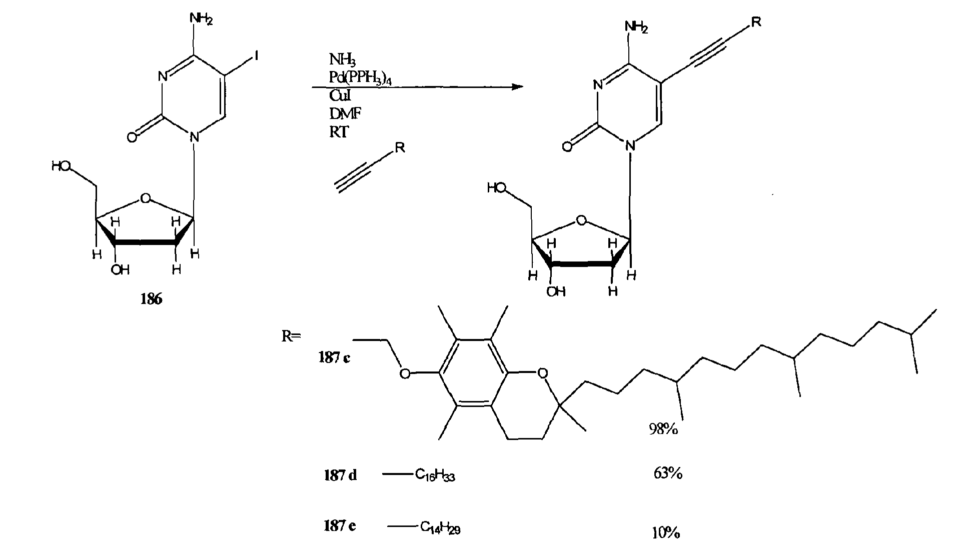

Using a Pd catalyst system the synthesis of 188 d was achieved at 56% yield:

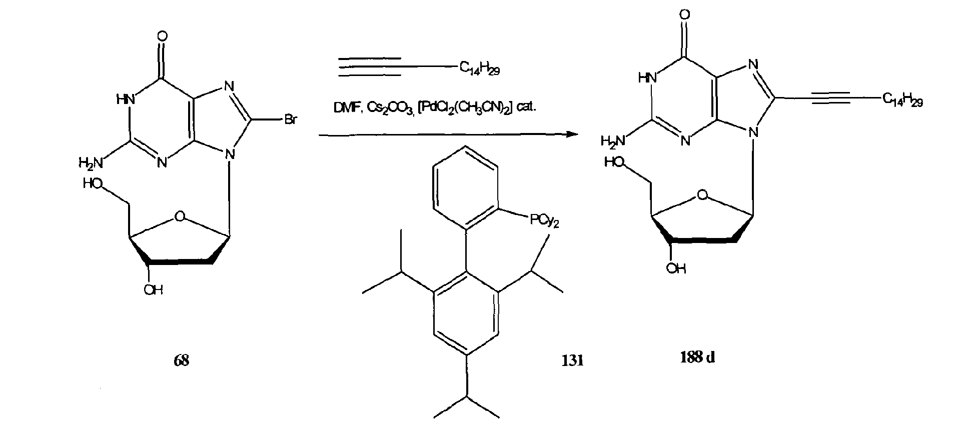

Also a series of Stille coupling reactions were performed on protected uridine:

In analogy to these reactions, also a reaction was performed using a lipophilic fluorescent 9,9-dialkyl substituted bromo-fluorene:

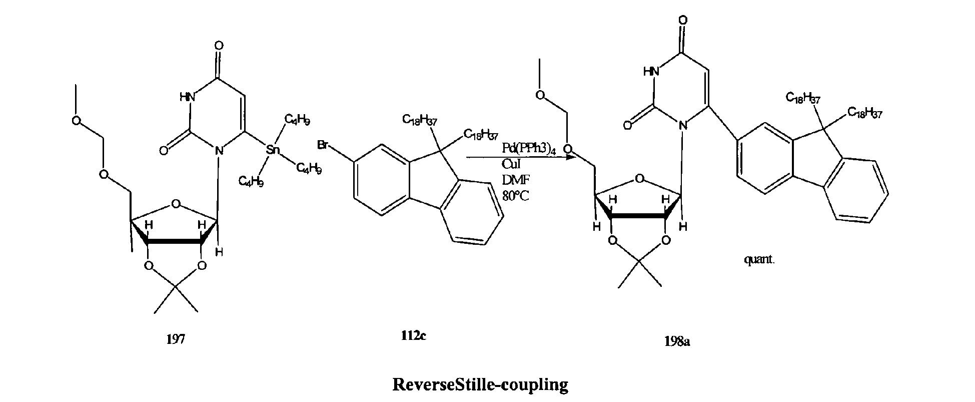

The product was obtained in a quantitative manner. Because of the high toxicity of tin organylen, the product was deprotected and cleaned up in several steps: 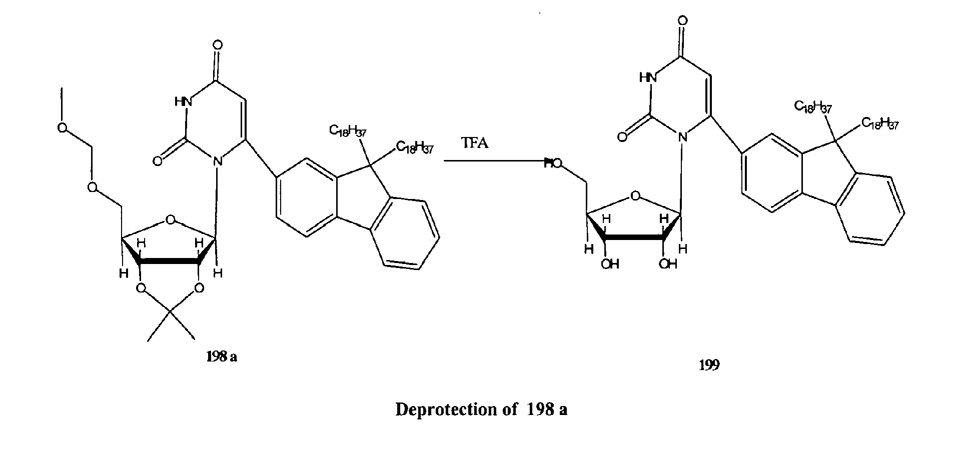 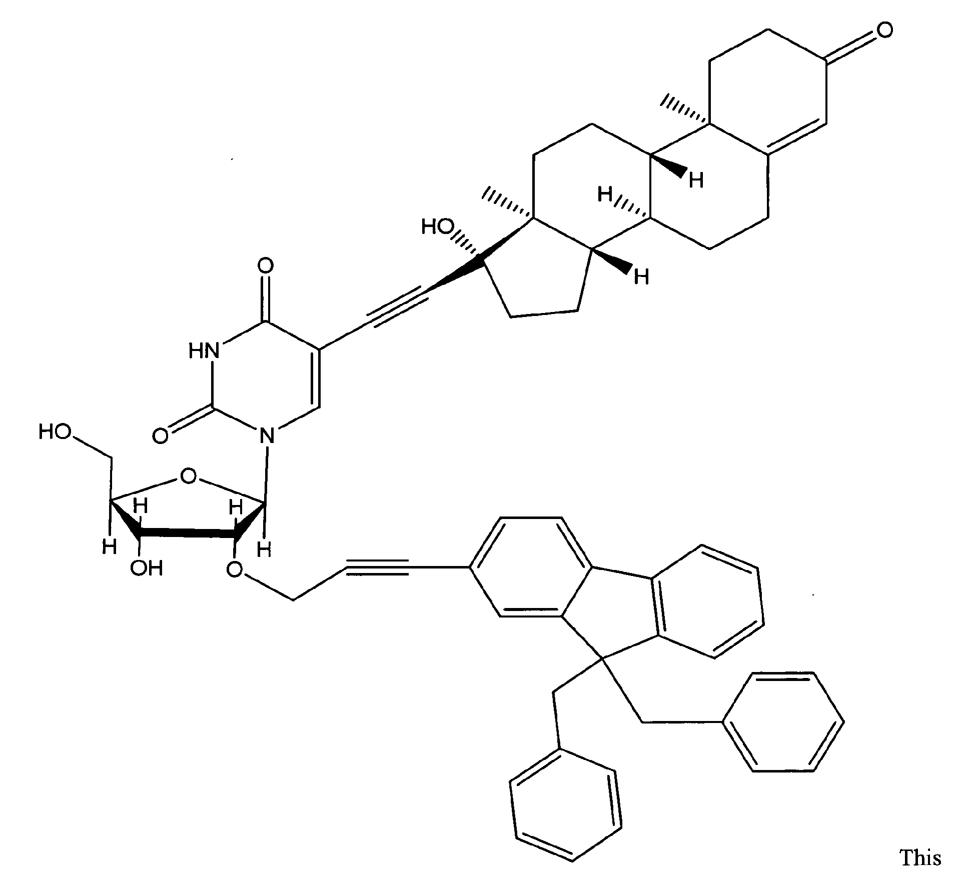 compound was synthesised from 5-iodouridine by first introduction of the steroidal alkynyl rest in position 5 of the uracil ring, protection of the 3'and 5'OH group by tetraisopropylsilyl dichloride, alkylation of the 2'-OH group by the fluorenylpargyl bromide and deprotection of the 3' and 5'-OH groups by ammonium fluoride.

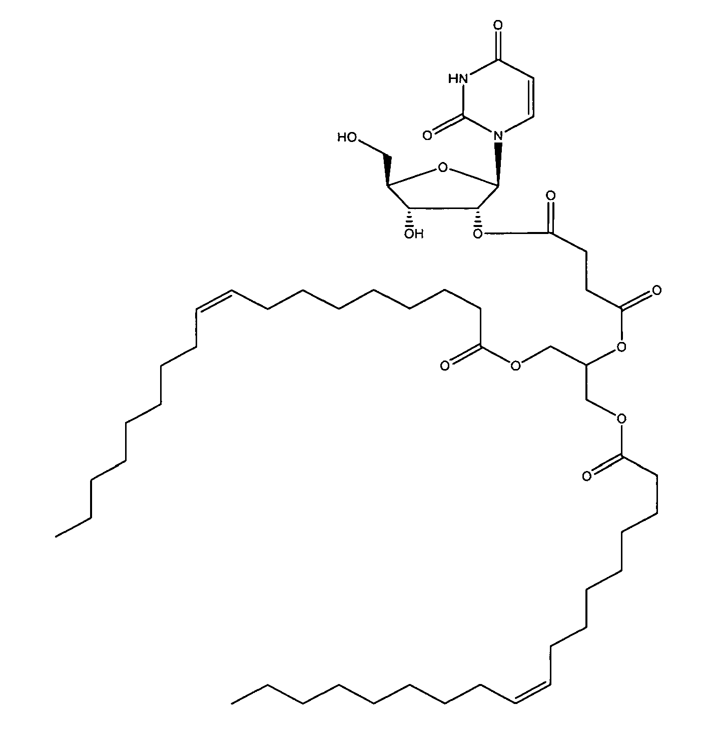

This compound was synthesized starting from uridine by protecting the 3'and 5-position by di(t-butylsilyldichloride and was acylated in the 2'-position by the corresponding succinate in the presence of N-ethyl-N'-dimethylaminopropylcarbodiimide and 4-dimethylaminopyridine.

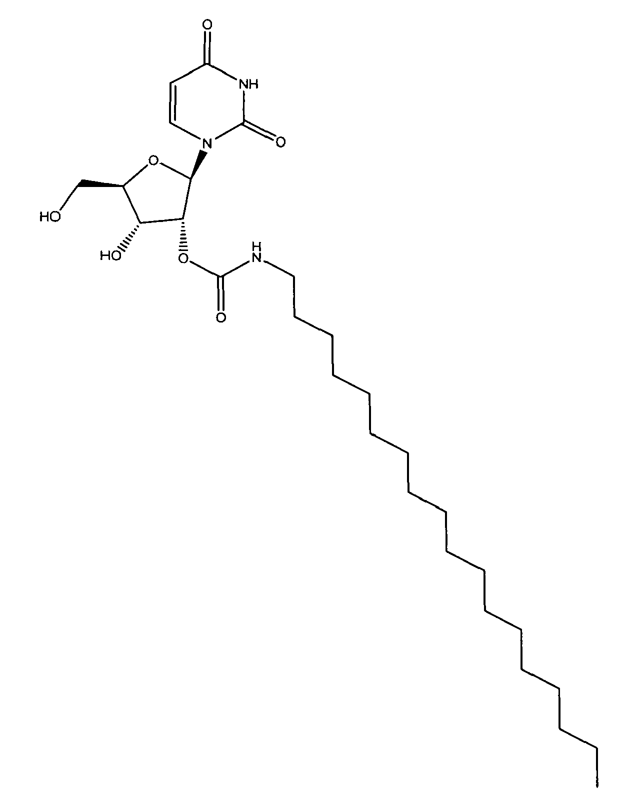

This compound was synthesized starting from uridine by protection of the 3',5'-OH groups by di(t-butyl)silyldichlorid, acylating the 2'-OH group with carbonyldiimidazole and final reaction with dodecylamine.

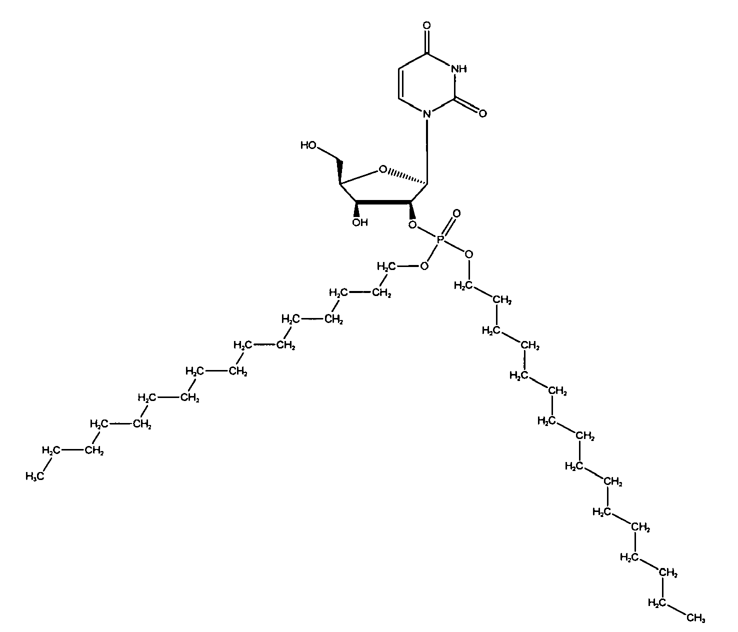

This compound was synthesized starting from uridine by protecting the 3'and 5-position by di(t-butylsilyldichloride and was phosphorylated in the 2'-position by N-diisopropyl phosphoramdious acid dihexadecyl ester in the presence ot tetrazole. The resulting phosphate was oxidized to the phosphate with m-chloroperbenzoic acid and deprotected with ammonium fluoride.

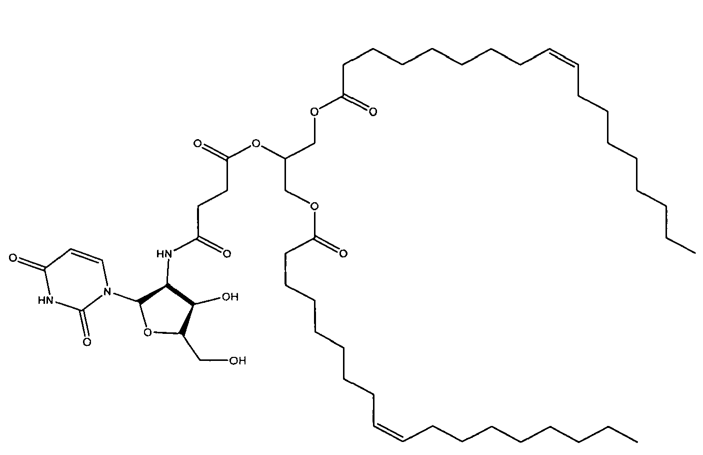

This compound was synthesized starting from 2'-amino-2'-desoxy-uridine protected with 4,4'-dimethoxytrityl in position 5' and trimethylsilyl in position 3'. Acylation with 4-chloro-4-oxo-butanoic acid 2-(dodecoyloxy)-1-[(dodecoyloxy)methyl]ethyl ester in tetrahydrofurane in the presence of sodium acetate and deprotection with 80% aqueous acetic acid leads to the product.

### References

[1] A. S. Boutorin, L. V. Gus'kova, E. M. Ivanova, N. D. Kobetz, V. F. Zarytova, A. S. Ryte, L. V. Yurchenko, V. V. Vlassov, FEBS Lett. 1989, 254 129-132.
[2] R. G. Shea, J. C. Marsters, N. Bischofberger, Nucleic Acids Res. 1990, 18 3777-3783.
[3] C. MacKellar, D. Graham, D. W. Will, S. Burgess, T. Brown, Nucleic Acids Res. 1992, 20 3411-3417.
[4] J. M. Tomkins, K. J. Barnes, A. J. Blaker, W. J. Watkins, C. Abell, Tetrahedron Lett. 1997, 38691-694.
[5] S. Bonaccio, P. Walde, P. L. Luisi, J. Phys. Chem. 1994, 98 6661-6663.
[6] A. M. Krieg, J. Tonkinson, S. Matson, Q. Zhao, M. Saxon, L. M. Zhang, U. Bhanja, L. Yakubov, C. A. Stein, Proc. Natl. Acad. Sci. U.S.A. 1993, 90 1048-1052.
[7] C. Lorenz, P. Hadwiger, M. John, H. P. Vornlocher, C. Unverzagt, Bioorg. Med. Chem. Lett. 2004, 14 4975-4977.
[8] J. Soutschek, A. Akinc, B. Bramlage, K. Charisse, R. Constien, M. Donoghue, S. Elbashir, A. Geick, P. Hadwiger, J. Harborth, M. John, V. Kesavan, G. Lavine, R. K. Pandey, T. Racie, K. G. Rajeev, I. Rohl, I. Toudjarska, G. Wang, S. Wuschko, D. Bumcrot, V. Koteliansky, S. Limmer, M. Manoharan, H. P. Vornlocher, Nature. 2004, 432 173-178.
[9] W. Flasche, C. Cismas, A. Herrmann, J. Liebscher, Synthesis 2004, 2335-2341.
[10]S. R. LaPlante, E. A. Boudreau, N. Zanatta, G. C. Levy, P. N. Borer, J. Ashcroft, D. Cowburn, Biochemistry. 1988, 27 7902-7909.
[11]J. Korlach, P. Schwille, W. W., G. W. Feigenson, Proc. Natl. Acad. Sci. U.S.A. 1999, %20;96 8461-8466.
[12]C. Dietrich, L. A. Bagatolli, Z. N. Volovyk, N. L. Thompson, M. Levi, K. Jacobson, E. Gratton, Biophys.J. 2001, 80 1417-1428.
[13]K. Bacia, P. Schwille, T. Kurzchalia, Proc. Natl. Acad. Sci. U.S.A. 2005, 102 3272-3277.
[14]P. Williamson, K. Mattocks, R. A. Schlegel, Biochim. Biophys. Acta. 1983; 732 387-393.
[15]R. G. Parton, A. A. Richards, Traffic. 2003, 4 724-738.
[16]M. J. Hope, M. B. Bally, G., P. R. Cullis, Biochim. Biophys. Acta 1985, 812 55-65.
[17]M. Angelova, S. Soleau, P. Meleard, J. F. Faucon, P. Bothorel, Prog. Lipid Res. 1992, 89 127-131.
[18] L. Mathivet, S. Cribier, P. F. Devaux, Biophys. J. 1996, 70 1112-1121.

## Claims

1. An oligonucleotide having formula 1 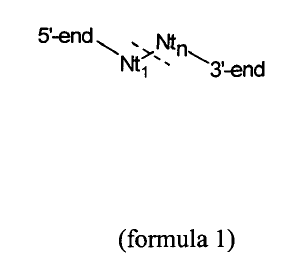 wherein
n denotes the total number of mononucleotides being present in said oligonucleotide, with n being ≥2,
the symbol " 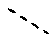 " signifies that there may be Nt₂, Nt₃ etc. between Nt₁ and Ntₙ, if n>2,
Nt₁, ..., Ntₙ are the same or different representing mononucleotides of the general formulae 2, 3, 4, or 5, 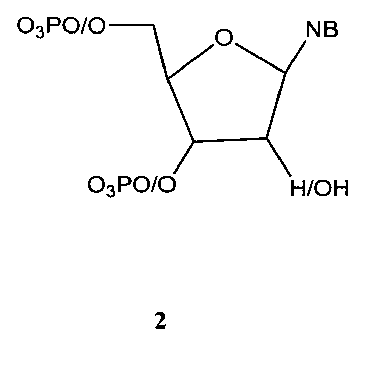 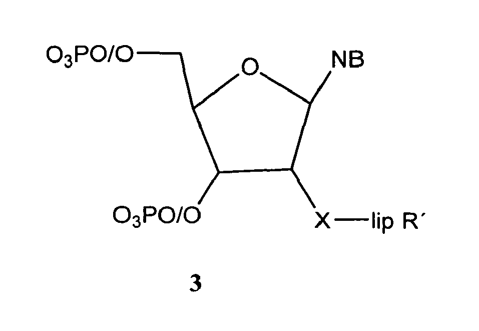 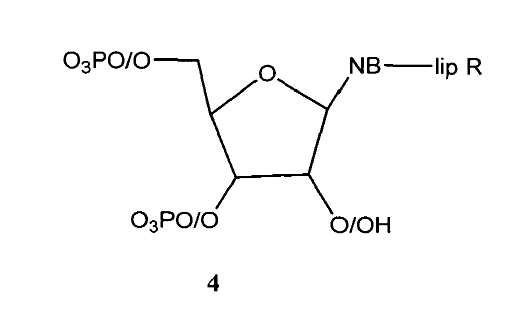 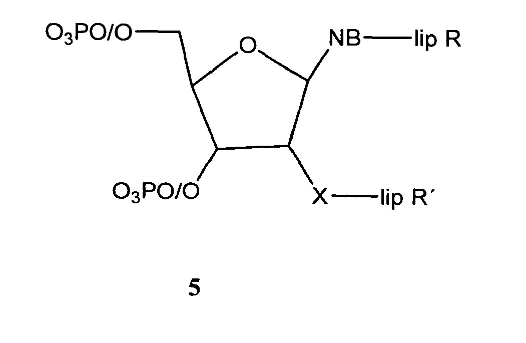 wherein at least one of Nt₁, ..., Ntₙ is a mononucleotide of formula 3, 4 or 5,
wherein X is O, N, S, phosphate or a single bond,
wherein lip R and lip R' are lipophilic moieties selected from the group comprising
fatty acyl groups having 6-30 C-atoms,
branched, unbranched, saturated or unsaturated open-chain or cyclic hydrocarbon groups having at least 10 carbon atoms,
branched, unbranched, saturated or unsaturated open-chain or cyclic hydrocarbon groups having at least 10 carbon atoms and one or several heteroatoms, like O, N or S,
terpenoid groups, such as sesquiterpenoids, diterpenoids, sesterterpenoids, triterpenoids, tetraterpenoids and polyterpenoids,
steroid groups,
lipid groups, such as diacylglycerol, wherein the two acyl chains are the same or different and have 6-30 C atoms, and phosphatidylcholines wherein the two acyl chains are the same or different and have 6-30 C-atoms, and phosphatidylethanolamines, wherein the two acyl chains are the s ame or different and have 6-30 C-atoms, and phosphatidyl serines, wherein the two acyl chains are the same or different and have 6-30 C-atoms, sphingolipids, and tocopherol groups,
wherein, in the case of lip R, said lipophilic moiety has a terminal C-C-triple bond, double bond or single bond and is linked to said NB via a terminal C of said triple bond, double bond or single bond, or wherein said lipophilic moiety is linked to said NB via a substituted or unsubstituted aromatic ring, preferably a benzene, a fluorene, an anthracene, more preferably a fluorescent aromatic ring, said aromatic ring forming part of lip R,
wherein, in the case of lip R', said lipophilic moiety is coupled directly or via -O- or via -S- or via -N- or via phosphate to the pentose ring,
wherein NB is a nucleobase selected from the group comprising purine bases and pyrimidine bases, such as adenine, guanine, cytosine, uracil, thymidine, 7-methylguanine, 5-methylcytosine, hypoxanthine, 4-thiouracil, 5-hydroxymethylcytosine, N²-methylguanine, N⁶-methyladenine, pseudouracil,
and wherein Nt₁ being the 5'-terminal mononucleotide has an OH- group or a phosphate group in the 5'-position and Ntₙ being the 3'-terminal mononucleotide has an OH-group or a phosphate group in the 3'-position, instead of the O indicated in these positions in formulae 2, 3, 4 and 5.

2. The oligonucleotide according to claim 1, wherein lip R or lip R' are -C≡C-C₁₂H₂₅, - C=C-tocopheryl, -C≡C-C₁₆H₃₃, decynyl, undecynyl, dodecynyl, tridecynyl, tetradecynyl, hexadecynyl, octadecynyl, eisosynyl, or steroidylethynyl

3. The oligonucleoctide according to claim 2, wherein lip R is -C≡C-C₁₂H₂₅, -C≡C-tocopheryl, -C≡C-C₁₆H₃₃, decynyl, undecynyl, dodecynyl, tridecynyl, tetradecynyl, hexadecynyl, octadecynyl, eisosynyl or steroidylethynyl.

4. The oligonucleotide according to any of the foregoing claims, wherein n is selected from the range from 2 to 60.

5. The oligonucleotide according to claim 4 comprising (n-1) mononucleotides according to formulae 3, 4 or 5.

6. The oligonucleotide according to claim 4, having n>2 and comprising at least 2, 3, 4, 5, ... or (n-2) mononucleotides according to formulae 3, 4 or 5.

7. The oligonucleotide according to claim 4, comprising n mononucleotides according to formulae 3, 4 or 5.

8. A nucleic acid duplex comprising in one strand an oligonucleotide according to any of claims 1-7.

9. The nucleic acid duplex according to claim 8 which is siRNA.

10. A nucleic acid vector comprising an oligonucleotide according to any of claims 1-7 or a nucleic acid duplex according to any of claims 8-9.

11. A lipid membrane comprising an oligonucleotide according to any of claims 1-7 or a nucleic acid duplex according to any of claims 8-9 or a nucleic acid vector according to claim 10.

12. The lipid membrane according to claim 11 having one or several lipid-disordered domains

13. The lipid membrane according to claim 11 having a lipid-ordered structure.

14. A method of synthesis of an oligonucleotide according to any of claims 1-7, wherein at least one of Nt₁, .., Ntₙ is a mononucleotide of formula 4, comprising the steps
- providing a mononucleoside of formula 10 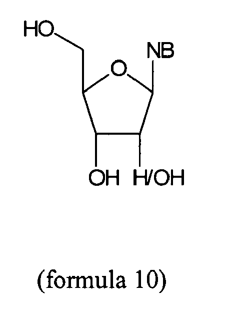 NB being as defined in claim 1 , wherein the OH-groups, if present in the 2' position, the OH-groups in the 3' and/or 5'-positions may be and, if desired, are protected by protecting groups, such as silyl groups, acetonides, benzyl groups, acyl groups, benzylidene, triarylmethyl,
- providing
either a compound of formula 6
R₁-C≡C-Z (formula 6)
R₁-C≡C representing lip R as defined in claim 1,
Z being hydrogen, a metal or boron or a leaving group such as a halide or a triflate,
or a compound of formula 7
R₁-CH=CH-Z (formula 7)
R₁-CH=CH representing lip R as defined in claim 1, Z being hydrogen, a metal or boron or a leaving group such as a halide or a triflate,
or a compound of formula 8
R₁-CH₂-CH₂-Z (formula 8)
R₁-CH₂-CH₂ representing lip R as defined in claim 1,
Z being hydrogen, a metal silica, phosphorus or boron or a leaving group such as a halide or a triflate,
or a compound of formula 9
Aryl-Z (formula 9)
Aryl representing lip R as defined in claim 1, wherein lip R is a lipohilic moiety having a substituted or unsubstituted aromatic ring, preferably a benzene, a fluorene, an anthracene, more preferably a fluorescent aromatic ring, Z being hydrogen, a metal or boron or a leaving group such as halide or triflate,
- reacting said nucleoside having formula 10 as defined above with said compound of formula 6, 7, 8 or 9, in the presence of a metal catalyst, such as a Pd catalyst, a Ni catalyst or a Fe catalyst and/or a copper salt, to form a mononucleoside according to formula 11, 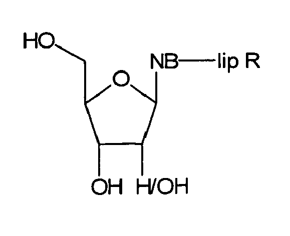 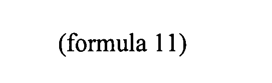 lip R and NB being as defined in claim 1, wherein the OH-groups if present in the 2' position, the OH-groups in the 3' and/or 5'-positions may be and, if desired, are protected by protecting groups such as silyl groups, acetonides, benzyl groups, acyl groups, benzylidene, triarylmethyl,
- using said mononucleoside in solid or liquid phase oligonucleotide synthesis, to form an oligonucleotide according to any of claims 1-7, wherein at least one of Nt₁, .., Ntₙ is a mononucleotide of formula 4.

15. A method of synthesis of an oligonucleotide according to any of claims 1-7, wherein at least one of Nt₁, .., Ntₙ is a mononucleotide of formula 3, comprising the steps
- providing a mononucleoside of formula 14 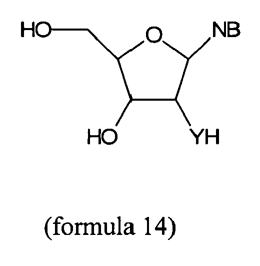 NB being as defined in claim 1, Y being one of O, NH, NAlkyl, S, wherein the OH-groups in the 3' and/or 5'-positions may be and, if desired, are protected by protecting groups such as silyl groups, benzyl groups, acyl groups, benzylidene, triarylmethyl,
- providing an acylation reagent having lip R' attached or an alkylating reagent having lip R' attached, said acylation reagent having the formula lip R'-COZ', Z' being a leaving group, such as OH, halogen, acyloxy, imidazole, said alkylating reagent having the formula Z-lip R', Z being a leaving group such as OH, halogen, a sulfonate, such as trifluoromethanesulfonate, tosylate, benzoylsulfonate, or providing a heterocumulene of the formula X=C=N-lip R' with X = O, S, NAlkyl, NAryl, or providing a phosphorylating reagent having lip R' attached, said heterocumulene being preferably an isocyanate, isothiocyanate or a carbodiimide, and
- reacting said mononucleoside of formula 14 with said acylation reagent, alkylating reagent, heterocumulene, or phosphorylating reagent, in the presence of a metal catalyst, such as a Pd catalyst, a Fe catalyst or a Ni catalyst and/or a copper salt, to form a mononucleoside according to formula 12 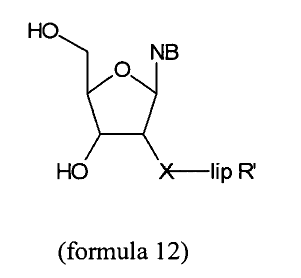
- using said mononucleoside in solid or liquid phase oligonucleotide synthesis, to form an oligonucleotide according to any of claims 1-7, wherein at least one of Nt₁, .., Ntₙ is a mononucleotide of formula 3.

16. A method of synthesis of an oligonucleotide according to any of claims 1-7, wherein at least one of Nt₁, .., Ntₙ is a mononucleotide of formula 5 comprising the steps
- providing a mononucleoside of formula 10 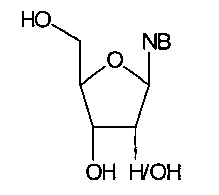 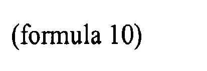 NB being as defined in claim 1,
- attaching, in any order, a lip R moiety at the nucleobase by a reaction for attaching such moiety at the nucleobase as defined in claim 14, and a lip R' moiety at the 2'-position of the pentose by a reaction for attaching such moiety at the 2' position as defined in claim 15, to form a mononucleoside according to formula 13 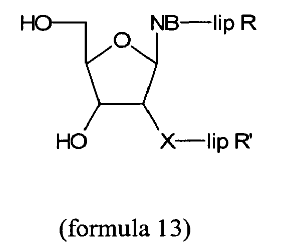
wherein the OH-groups in the 3' and/or 5'-positions may be and, if desired, are protected by protecting groups such as silyl groups, benzyl groups, acyl groups, benzylidene, triarylmethyl,
- using said mononucleoside in solid or liquid phase oligonucleotide synthesis to form an oligonucleotide according to any of claims 1-7, wherein at least one of Nt₁, .., Ntₙ is a mononucleotide of formula 5.

17. Use of an oligonucleotide according to any of claims 1-7 for incorporation into a nucleic acid duplex, preferably small interfering RNA (siRNA).

18. Use of an oligonucleotide according to any of claims 1-7 and/or a nucleic acid duplex according to any of claims 8-9, and/or a nucleic acid vector according to claim 10 for facilitating the uptake of nucleic acids into cells.

19. Use of an oligonucleotide according to any of claims 1-7 and/or a nucleic acid duplex according to any of claims 8-9, and/or a nucleic acid vector according to claim 10 for structuring a lipid membrane, preferably a lipid bilayer membrane, and/or for establishing/immobilizing/arranging a chemically reactive complex in a lipid membranes ("nanoreactor"), preferably different enzymes involved in the same reaction (cascade) which are arranged in close neighborhood on a lipid membrane, and/or for structuring surfaces of nucleic acid chips having lipophilic surfaces, and/or for establishing molecular receptors in lipid membranes.
